# EUROPEAN PATENT APPLICATION

(11) **EP 2 161 019 A1**
(43) Date of publication of application: **10.03.2010**
(21) Application number: 08015689.6
(22) Date of filing: 05.09.2008
(51) Int. Cl.: A61K 9/50

(54) **Prolonged release multiparticulate pharmaceutical composition comprising paliperidone**

(71) Applicant: KRKA, D.D., Novo Mesto, 8501 Novo mesto (SI)
(72) Inventor: Vrecer, Franc, 8351 Straza pri Novem mestu (SI); Pisek, Robert, 1000 Ljubljana (SI); Preskar, Maja, 8270 Krsko (SI); Kapele, Tomaz, 8340 Crnomeij (SI)
(74) Representative: HOFFMANN EITLE

(57) **Abstract**

The present application pertains to pharmaceutical compositions comprising paliperidone or a pharmaceutically acceptable salt thereof as the active substance, wherein the pharmaceutical composition is in the form of pellets comprising a core particle, optionally one or more intermediate coatings, at least one prolonged release coating and optionally one or more outer coatings, in this order, wherein the active substance is present in the core particle, and wherein the pellets have a maximum diameter of 2 mm or less and exhibit favourable release characteristics.

## Description

### Field of the invention

The present invention is directed to a composition and a method for the preparation of prolonged release pharmaceutical compositions comprising paliperidone or a pharmaceutically acceptable salt thereof.

The invention is also directed to a sustained release pharmaceutical composition comprising paliperidone or a pharmaceutically acceptable salt thereof, wherein composition can be in the form of coated pellets or capsules for once-a-day or twice-a-day oral administration.

### Background of the Invention

Schizophrenia is a chronic, severe, and disabling psychotic disorder characterized by extreme disturbances of cognition and thought, affecting language, perception and sense of self. It is a mental illness with a lifetime estimated risk of 1%.
Schizophrenia is characterized by positive symptoms (auditory hallucinations, disorganized or bizarre thoughts, delusions and irrational fears) and negative symptoms of social withdrawal, poor motivation, poverty of speech, apathy and lack of energy.

One of the key issues in schizophrenia management is adherence with treatment. It is estimated that nearly half of outpatients with schizophrenia are noncompliant or only partially compliant with their therapy during the first year after hospital discharge. The consequences of non-adherence or partial adherence in the majority of cases include relapse and re-hospitalization. Medication non-adherence is especially difficult in this population because of both behavioral (e.g., denial of disease) and cognitive (e.g., forgetting to take medication) issues.

The treatment of schizophrenia is multifactorial, with antipsychotic medications comprising a major part of treatment.
Chlorpromazine and other first-generation antipsychotics (or typical antipsychotics) antagonize the dopamine D₂-like class of receptors. Although effective against psychosis, they do not improve and may even exacerbate the negative symptoms of schizophrenia and are associated with dose-limiting extrapyramidal symptoms (EPS). The second-generation dual-action dopamine and serotonine D₂/5-HT_{2A} receptor blockers (or atypical antipsychotics) retain the antipsychotic effect of the typical antipsychotics, but show a much reduced propensity to cause EPS, and also may improve negative symptoms of schizophrenia.

Paliperidone represents the most recent atypical antipsychotic indicated for the short- and long-term treatment of schizophrenia.
Paliperidone, a benzisoxazole derivative and the principal active metabolite of risperidone, is commonly referred to as 9-hydroxyrisperidone.
Paliperidone, the chemical name of which is (±)-3-[2-[4-(6-fluoro-1,2-benzisoxazol-3-yl)-1-piperidinyl]ethyl]-6,7,8,9-tetrahydro-9-hydroxy-2-methyl-4*H*pyrido[1,2-a]pyrimidin-4-one, was first disclosed in EP 0 368 388 B1.

Prolonged release formulations offer the possibility of reducing dosage regimes for drugs, especially for those drugs administered orally to patients, by prolonging the time period during which pharmacologically effective levels of the active substance are present in the body. Prolonged release formulations thereby result in a better assurance of compliance, reduction of severity and frequency of side effects, since the drug level in the blood is more constant, and drug level fluctuations associated with conventional immediate release formulations administered several times a day are avoided.

The following documents are directed to osmotic-controlled release dosage forms (formulated by using OROS drug delivery technology) that in general utilize osmotic pressure to generate a driving force for imbibing fluid into a compartment formed, at least in part, by a semipermeable membrane that permits free diffusion of fluid but not drug or osmotic agent(s), if present.

WO 2004/010981 relates to dosage forms and methods for providing a substantially ascending rate of release of paliperidone. Moreover, the patent is concerned with a dosage form comprising a capsule shaped tablet core containing a plurality of layers wherein the paliperidone is contained in at least one layer and at least one other layer comprises a suitable fluid-expandable polymer; (b) a semipermeable membrane surrounding the capsule shaped tablet core to form a compartment having an osmotic gradient to drive fluid from an external fluid environment contacting the semipermeable membrane into the compartment; and (c) an orifice formed through the semipermeable membrane and into the capsule shaped tablet core to permit paliperidone to be released from within the compartment into the external fluid environment.

OROS dosage forms are further disclosed in the following documents WO 2005/048952, WO 2006/085856, WO 2006/101815, WO 2007/016388, WO 2007/081736, WO 2007/044234.

Disadvantages to osmotic pump dosage forms include complex manufacture and the use of harsh solvents in their preparation.

Robert Conley, et al. (Clinical spectrum of the osmotic-controlled release oral delivery system (OROS), an advanced oral delivery form, Current medical research and opinion 2006, 22(10), pp. 1879-1892) describe that the OROS tablet is excreted in the feces and this may be problematic in some patient groups, for example, schizophrenia patients, where the excretion of foreign bodies may be disturbing. Some patients, as with any oral medication, may have difficulty swallowing the tablet and this may limit its utility in this population of patients.

Another drawback of osmotic pump dosage forms is in the fact that such dosage forms are due to their size transported along gastrointestinal tract slower and less predictable than multiparticulate dosage forms such as pellets wherein individual particles i.e. pellets have a maximum diameter of less than 2 mm. It is known, that particles with a maximum diameter of less than 2 mm are transported from the stomach like a liquid so their transition through gastrointestinal tract is much more predictable what results in a more predictable release of active substance from the dosage form and so more controllable therapy in patients.

It is known that achieving prolonged release of active substance from small coated particles is a difficult task due to the very high surface area of particles which contributes to fast release of active substance from the core particles.

### Summary of the Invention

The present invention solves the above mentioned problems and provides a prolonged release formulation of active substance paliperidone or its pharmaceutically acceptable salts that permits a predictable passage through the gastrointestinal tract whilst allowing slow and predictable release of the active substance.

The present inventors have surprisingly found that the above-mentioned benefits can be attained by discovering that the prolonged release of active substance paliperidone or its pharmaceutically acceptable salts from multiparticulate dosage forms comprising particles such as pellets having a maximum diameter of less than 2 mm can be assured by coating of core particles containing paliperidone or its pharmaceutically acceptable salts having a maximum diameter of less than 2 mm with at least one prolonged release coating decreasing the release rate of active substance from the core.

The formulation of the present invention is specified in appended Claim 1. Preferred embodiments of this formulation are specified in appended Claims 2 to ...

### Brief Description of the Figures

Figure 1: *In vitro* dissolution profile of active substance from formulation Example 7 in 500 ml of 0.1M HCl at 37°C in an Apparatus 1 - basket (Ph. Eur. or USP, 100 rpm).
Figure 2: *In vitro* dissolution profile of active substance from formulation Example 7 in 500 mL USP buffer pH 6.8 at 37°C in an Apparatus 1 - basket (Ph.Eur. or USP, 100 rpm).

### Detailed Description

### 1. Definitions

"Active substance" in the context of this invention means paliperidone or a pharmaceutically acceptable salt thereof. The active substance contained in the pharmaceutical composition of the present invention may be present in an unionized form or in the form of a salt, hydrate or solvate thereof. It may further be present in a crystalline or non-crystalline form such as a polymorphic, pseudopolymorphic or amorphous form. The term "active substance" should be understood to include any such salt, in crystalline or noncrystalline form including all polymorphic and/or solvated forms of the respective active substance.

"Prolonged release" in the context of this invention means that the pharmaceutical composition exhibits a single release kinetics with a release profile such that after 2 hours less than 25% of the active substance originally contained in a particle type is released, after 12 hours 25 to 65% of the active substance contained in a particle type is released, and after 24 hours more than 65% of the active substance contained in a particle type is released after dissolving the dosage formulation in 500 ml USP buffer pH 6.8 at 37°C in an Apparatus 1 - basket (Ph.Eur. or USP, 100 rpm). Unless stated otherwise, all percentages given in this patent application are by weight.
In order to optimize active substance absorption and consequently its plasma profile, the pharmaceutical composition according to present invention can have a "lag time", meaning the time period during which less than 5% of active substance is released from the composition. The lag time, determined in vitro using 500 ml of 0.1M HCl at 37°C in an Apparatus 1 - basket (Ph. Eur. or USP, 100 rpm), of the composition of the present invention is typically less than 4 hours, preferably of less than 3 hours more preferably of less than 2 hours and most preferably between 1 and 2 hours.

The term "average particle size" as used herein refers to the volume mean diameter of particles. The diameter and volume mean diameter can be determined by laser light scattering using e.g. a Malvern-Mastersizer Apparatus MS 2000. Particle sizes are determined by measuring the angular distribution of laser light scattered by a homogeneous suspension of particles. The particles to be subjected to the particle size measurement are first suspended in appropriate non-polar dispersant and then subjected to a size determination in a Malvern Mastersizer MS 2000 instrument. Usually, 100-800 mg of substance are dispersed in 5-10 ml of dispersant.

The "maximum diameter" of particles is determined by vibrational sieve analysis. Sieves with defined size of holes (2, 1.4, 1, 0.84 mm) are used in the analysis. The maximum diameter specifies the maximum hole size that is passed by all particles.

The term "substantially spherical particle" according to present invention means a particle wherein the ratio of larger and smaller diameter of the particle is in the range of 1.0 to 1.6, preferably 1.0 to 1.4.

The term "insoluble" is used in the present invention to specify substances, which, according to USP/ Ph. Eur. General Notice/ Solubility; are insoluble or practically insoluble, namely having a solubility of 1g substance or less in 10.000 ml of fluid.

### 2. Coated Particles

The present invention provides for a solid pharmaceutical composition comprising coated particles comprising paliperidone or a pharmaceutically acceptable salt thereof as an active substance and wherein said solid pharmaceutical composition provides for prolonged release of said active substance. The coated particles comprise a core particle containing active substance and optionally other excipients and at least one coating having a prolonged release effect onto the release of active substance from the core. The core particle is preferably in substantially spherical shape and has a maximum diameter of less than 2 mm.

In order to assure good stability of the active substance incorporated into coated particle, the moisture content in the particles is preferably controlled and fixed in the range 0.5 to 3 weight%, preferably 0.75 to 2.5 weight% (determined by loss on drying by halogen dryer such as Mettler Toledo halogen dryer HR73 at 85°C for 20 min).

### 3. Active Substance

Paliperidone can generally be prepared by any known process such as the processes described in EP 0 368 388 B1, WO 2008/024415, WO 2008/021345.

Paliperidone used in the present invention can be in any crystalline or noncrystalline form such as for example disclosed in WO 2008/021342 and IPCOM000167996D (http://www.priorartdatabase.com).

As paliperidone belongs to the group of low solubility active substances, it is important that the particle size, particle shape and specific surface area of the active substance are controlled. Average particle size of active substance of the present invention is in the range of 1 to 250 µm, preferably of 5 to 50 µm in order to achieve optimal process optimization and product characteristics.

The amount of active substance present in the pharmaceutical composition according to the present invention can be 0.5-25 mg, preferably 1-20 mg and most preferably 1.5-15 mg of paliperidone per final dosage form.

### 4. Core Particles

The present invention uses core particles containing the active substance.

Said core particles are preferably of substantially spherical shape. The coating of substantially spherical particles of small diameter in comparison to coating of nonspherical particles such as tablets results in a much more uniform thickness of the coating, due to the absence of sharp edges in case of spherical particles, where the thickness of the coating is usually lower than in case of flat or rounded surfaces, what results in more uniform release of an active substance from the composition what is beneficial for the patient.

The maximum diameter of the core particles is less than 1.6 mm, such that the particles after coating have a maximum diameter of 2 mm or less. Preferably, the maximum diameter of the core particles is less than 1.2 mm and most preferably the diameter of the core particles is in the range 0.2 to 1 mm.

In one embodiment of the present invention the core particles can be obtained by producing matrix core particles by methods known from the state of the art such as extrusion and spheronization, direct pelletization of powder mixture of the active substance and at least one pharmaceutically acceptable inactive ingredient selected from binder, diluent, prolonged release substance, surfactant, glidant, lubricant, disintegrant and/or antioxidant in pelletizer such as rotor pelletizing equipment such as those produced by Glatt GmbH or Freud or high shear mixer granulator with a pelletizing liquid such as water or aqueous solution of a binder. Matrix core particles in the present invention mean particles wherein the active substance and at least one matrix former selected from diluent, binder, prolonged release substance, surfactant, glidant, lubricant, disintegrant and/or antioxidant are homogenously distributed throughout the whole volume of the core particle.

In one embodiment of the present invention active substance and prolonged release substance may be granulated, followed by extrusion to form the matrix core particles mentioned above. Granulation can be performed by the state of the art processes and equipment using water, organic solvent or the mixture thereof as granulation solvent. Binders selected from water soluble polymers such as hypromelose having viscosity of 2w/vol% aqueous solution at 20°C of less than 20 mPas, preferably less than 15mPas, hyprolose, povidone with K value in the range 5 to 100 such as povidone K30 and povidone K90, copovidone, methylcellulose, polyvinyl alcohol, hydroxyethyl cellulose, polyethyleneglycol can optionally be used in the preparation of granules. Binders selected from hydrophilic polymers can be included into the granulate as powder added to the powder mixture of active substance and prolonged release substance or as dissolved in the granulation liquid. Further excipients selected from diluents, glidants, surfactants, disintegrants, lubricants, antioxidants, water insoluble lipid-based excipients such as esters of fatty acids with 10-18 C atoms with aliphatic alcohols having 8-18 C atoms, mono, di and/or triesters of glycerol with fatty acids with 10 to 26 C atoms such as glyceryl palmitostearate, glyceryl behenate, waxes, fatty acids such as stearic acid and palmitic acid, higher aliphatic alcohols such as stearol, palmitol or the mixtures thereof can be used in a preferred embodiment to optimize the granulation process and granule properties.

In another embodiment of the present invention core particles containing active substance, a binder and optionally further excipients can be obtained by melt pelletization techniques known from the state of the art such as hot melt extrusion and spheronization, hot melt pelletization in high shear mixers wherein the binder having melting point below 100°C, preferably below 80°C, more preferably below 70°C and most preferably below 60°C can be selected from water soluble excipients such as poloxameres, fatty acid esters of macrogolglycerides such as Gelucire types, sugar esters with fatty acids with 10-22 C atoms and water insoluble excipients such as glyceryl behenate, glyceryl palmitostearate, waxes or the like. Further excipients can be used selected from diluents, prolonged release substances, lubricants, surfactants, disintegrants, glidants, antitacking agents and antioxidants.

In another embodiment of the present invention the core particles containing the active substance can be obtained by coating of inert particles with a dispersion containing active substance dissolved, emulsified or suspended in an appropriate liquid vehicle such as water, organic solvents such as alcohols with 1-4 C atoms, such as methanol, ethanol, isopropanol, ketones such as acetone and or mixture thereof and optionally further inactive ingredients selected from diluent, binder, surfactant, disintegrant, glidant, lubricant, antitacking agent and antioxidant. Coating can be performed by the state of the art processes and equipment such as coating pan, fluid bed coater with top, bottom or tangential position of spray nozzle. In a preferred embodiment of the present invention coating of inert particles with active substance containing layer can be performed by the process known from the state of the art such as powder layering wherein the powder mixture of active substance and optionally other excipients selected from diluent, disintegrant, binder, prolonged release substance, surfactant, lubricant, glidant and antioxidant is applied onto inert particles by simultaneous spraying of the binder solution in a coating equipment equipped with powder feeding device and separate spray nozzle for liquid spraying. In a further embodiment of the present invention active substance can be layered onto the inert cores by a process of hot melt coating using low melting binder described above. Active substance can be applied onto the inert cores as powder optionally admixed with further excipients selected from diluent, disintegrant, prolonged release substance, surfactant, lubricant, glidant and antioxidant with simultaneously spraying the melted binder or can be applied as dispersion such as solution or suspension in the melted binder together with further excipients selected from diluent, disintegrant, prolonged release substance, surfactant, lubricant, glidant and antioxidant. Coating can be performed by the state of the art coating processes and equipment selected from solid wall pan coater such as those produced by company IMA, fluid bed coater such as bottom spray equipment produced by Glatt GmbH, Aeromatic, Hüttlin and tangential spray equipment such as rotor processor produced by Glatt GmbH, Freud tangential spray equipment or the like.

### 5. Prolonged Release Coating

The prolonged release coating is formed using at least one prolonged release compound and optionally one or more further components.

The prolonged release substance can be selected from the group consisting of excipients which swell upon contact with physiological fluids, non-swellable polymers and insoluble excipients and can be used in the weight ratio to the total weight of coated particles in the range from 1 : 5 to 1 : 50, preferably from 1:7 to 1:30.

Swellable excipients (polymers) are defined by gelling index as disclosed in EP 661 045 B1, cf. paragraph [0032]. A method for determining the gelling index is described in paragraph [0031] of this document.

The excipients which swell upon contact with physiological fluids can be selected from hydroxypropylmethylcellulose of different viscosity and/or substitution grades having the viscosity of 2% solution in water (determined according to USP method) in the range of 50 to 250,000 mPas and having methoxyl group content in the range of 19 to 32 w/w% and hydropropyl group content in the range 7 to 12 w/w%, hydroxypropyl cellulose e.g. Klucel HF, HXF or MF types having the viscosity of 1% aqueous solution in the range of 1,000 to 4,000 mPas, hydroxycellulose phthalate, poly(ethyleneoxide) of molecular weight in the range of 1,000,000 to 7,000,000 e.g. Polyox WSR303, polylactic acid, xanthan gum, alginates, sodium and calcium carboxymethylcellulose, carrageenan (Carrageenan Iota, Kappa, Lambda), carbomer, carbopol (different product types e.g. Carbopol 971 P, Carbopol 71 G), methacrylic ester copolymers sold as Eudragit® NE, methylhydroxyethylcellulose, propylhydroxyethylcellulose, polyHEMA, methylcellulose, alginates and other swellable polymers.

The non-swellable polymer can be selected from the group consisting of water insoluble polymers such as for example ethyl cellulose of different viscosity types having a viscosity of 5w/vol% solution in mixture of toluene and alcohol in the range of 3-100 mPas (Ethocel), cellulose acetate propionate, cellulose acetate, poly(ethyl acrylate, methyl methacrylate, trimethylammonioethyl methacrylate chloride)1:2:0.1, sold as Eudragit® RS 100 poly(ethyl acrylate, methyl methacrylate, trimethylammonioethyl methacrylate chloride) 1:2:0.2 copolymer, commercially available as Eudragit® RL, polyvinylpyrrolidone acetate, Eudragit® RS PO, methacrylic ester copolymers (Eudragit NE), methylmethacrylate ethylacrylate copolymer (Kollicoat EMM), polyvinyl chloride, polyvinyl acetate, mixture of polyvinyl acetate and polyvinylpyrrolidone, commercially available as Kollidon SR or Kollicoat SR and polyethylene.

The insoluble excipient can be selected from lipid-based substances such as fatty alcohols with 10-18 C atoms such as stearol, palmitol, esters and ethers of fatty acids with alcohols such as glycerol, fatty alcohols such as mono, di and triglycerides with 10-18 C atoms in the fatty acid residue, waxes.

The core particles obtained according to any of above mentioned and described aspects of the present invention are coated with at least one coating having prolonged release effect onto the release rate of active substance from the core particles. Such coating can be applied as a dispersion of prolonged release polymer in water or organic solvent, wherein the dispersion of prolonged release polymer means that the polymer is dispersed (e.g. emulsified or suspended) in water or dissolved in appropriate organic solvent. In a special aspect of the present invention coating can be applied as a melt of a lipid-based substance having a melting point below 150°C, preferably below 100°C and even more preferably below 80°C such as synthetic and naturally occurring mixed glycerides, glyceryl behenate glyceryl palmitostearate. Further inactive ingredients can be used in such as such as plasticizers, anti-tacking agents, glidants, pore formers, pigments and colorants.

The prolonged release coating preferably has an average thickness within the range of 10 to 80 µm, preferably 15 to 60 µm. The average thickness of the individual coatings of coated particles is determined by scanning electron microscopy of the cross-section of particle by at least three measurements.

### 6. Further Components of the Pharmaceutical Composition

The diluent can be selected from the group consisting of water soluble excipients such as lactose, mannitol, sorbitol, dextran and water insoluble excipients such as crystalline cellulose including microcrystalline cellulose, starch, starch derivatives such as pregelatinized starch, earth alkali metal salts of phosphoric acid such as calcium hydrogen phosphate. Diluent can be present in the composition of coated particles in the range of 5 to 60 weight%, preferably 10 to 50 weight% calculated on the weight of the coated particles.

The disintegrant can be selected from the group consisting of crospovidone, starch, pregelatinised starch, sodium starch glycollate, microcrystalline cellulose, carboxymethylcellulose sodium (CMC-Na) or calcium (CMC-Ca), cross-linked CMC-Na, polacrilin potassium, low-substituted hydroxypropylcellulose or mixtures thereof and can be present in the composition in an amount of 0.5 to 15 weight%, preferably 1 to 12 weight% based on the weight of pharmaceutical composition.

The surfactant, if present, can be selected from the group consisting of anionic surfactants, ampholytic surfactants, nonionic surfactants and cationic surfactants. Surfactant can be present in the pharmaceutical composition in the range of 2 to 25 weight%, preferably 5 to 20 weight% calculated on the weight of active substance.

In a preferred aspect the anionic surfactant can be selected from the group consisting of alkali metal or earth alkali metal sulfonates (RSO₃⁻) or sulfates (ROSO₃⁻), potassium laurate, CH₃(CH₂)₁₀COO⁻K⁺, and sodium lauryl sulphate, CH₃(CH₂)₁₁SO₄⁻Na⁺. The most preferred anionic surfactant can be sodium lauryl sulphate.

In a preferred aspect the cationic surfactant can be selected from the group consisting of organic quaternary ammonium halides, R₄N⁺Cl⁻, cetrimide, a mixture consisting of tetradecyl (about 68%), dodecyl (about 22%), and hexadecyltrimethylammonium bromides (about 7%), as well as benzalkonium chloride, a mixture of alkylbenzyldimethylammonium chlorides of the general formula [C₆H₅CH₂N⁺(CH₃)₂R]Cl⁻, where R represents a mixture of alkyls from C₈H₁₇ to C₁₈H₃₇.

In a preferred aspect the ampholytic surfactant can be selected from sulfobetaines, RN⁺(CH₃)₂CH₂CH₂SO₃⁻, N-Dodecyl-N,N-Dimethylbetaine, C₁₂H₂₅N⁺(CH₃)₂CH₂COO⁻.

In a preferred aspect the nonionic surfactant can be selected form the surfactants containing hydroxyl or polyoxyethylene (-O-CH₂CH₂⁻)ₙ groups, and more preferably it can be selected from polyoxyethylated glycol monoethers, cetomacrogol, sorbitan esters (Spans) and polysorbates (Tweens), or from polyoxyethylene-polyoxypropylene blockcopolymers (poloxameres), or from the group of esters such as dioctyl sulphosuccinate and/or sucrose esters with fatty acids such as sucrose stearate or the like.

The composition according to the present invention may further comprise one or more antioxidants selected from the group consisting of alkyl gallates (e.g. dodecyl-, ethyl-, octyl-, propyl-gallate), butylated hydroxyanisole, butylated hydroxytoluene, tocopherols (e.g. alpha tocopherol), ascorbic acid palmitate, ascorbic acid, sodium ascorbate, potassium and sodium salts of sulphurous acid (e.g. bisulphites, metabisulphites, sulphites), flavonoides (rutin, quercetin, caffeic acid). The antioxidant can be present in the pharmaceutical composition in the weight ratio to active substance of 1 : 0.1 to 1:10, preferably 1:0.5 to 1:5.

Glidant can be selected from colloidal silicone dioxide.

Lubricant can be selected from the group consisting of glycerol behenate, earth alkali metal salts of stearates such as magnesium stearate, sodium stearyl fumarate or the like.

Anti-tacking agent can be selected from glyceryl monostearate and talc and can be used in concentration 10 to 50 weight% calculated on the weight of the coating.

Inert particles can be selected from neutral pellets made of saccharide such as lactose, sucrose and starch, neutral pellets made of microcrystalline cellulose. Average diameter of inert core can be in the range of 100 to 1000µm, preferably 400 to 850µm and can be present in the composition of the coated particles in concentration 15 to 70 weight%, preferably 20 to 60 weight%. The inert particle is preferably substantially spherical.

Pore formers can be selected from water soluble substances having molecular weight of less than 1,000 and having solubility of at least 1 g in 10 ml of water such as lactose, sucrose, fructose, mannitol, sorbitol, inositol, dextran, sodium chloride, citric acid, alkali metal salts of citric, carbonic, phosphoric and sulphonic acid such as sodium citrate, sodium bicarbonate, meglumine, poloxamere, sodium lauryl sulphate and from hydrophilic polymers such as polyethylene glycol with molecular weight in the range 400 to 10,000, low viscosity types of HPMC having viscosity of 2w/vol% aqueous solution at 20°C of less than 20 mPas or HPC, HEC, povidone with K values of less than 50, preferably of less than 35, copovidone. Pore formers can be included in the composition of the prolonged release coating in concentration 1 to 30 weight % preferably 2 to 20 weight% and most preferably 3 to 10 weight%, calculated on the dry weight of prolonged release coating.

Plasticizers can be selected from substances capable of decreasing the glass transition point (Tg) of polymer by at least 30°C such as alkyl esters of citric acid (e.g. triethylcitrate), phtalates, dibutylsebacate or the like in the concentration 5-50%, preferably 8-30%, more preferably 10-20% calculated on the weight of dry polymer.

Magnesium stearate, partial esters of glycerol with fatty acids with 10 to 18 carbon atoms such as glycerol monostearate (GMS) or talc can be selected as anti-tacking agents. Colloidal silicone dioxide can be used as a glidant. Metal oxides such as titanium dioxide and/or iron oxides can be selected as pigments.

### 7. Further Coatings

Optionally one or more layers of intermediate coating can be applied between the core and the prolonged release coating in order to improve the smoothness of the surface of core particles. The intermediate coating can comprise water soluble or water insoluble polymer and optionally other excipients selected from diluent, anti-tacking agent, antioxidant and glidant. The average thickness of any intermediate coating layer can be in the range of 5 to 80 µm, preferably 10 to 40 µm. Intermediate coating can represent 3 to 15, preferably 5 to 10 weight % of the total weight of coated pellets.

The particles coated with prolonged release coating can optionally be further coated with an overcoating in order to increase the mechanical strength of prolonged release coating in case of compression of particles into tablet or to improve physical and organoleptic properties of coated particles such as moisture resistance, colour, smoothness, taste, diminishing the permeability of gases such as oxygen. Overcoating is based on water soluble polymer, which can be present in an amount of at least 10% of the weight of the overcoating. In order to achieve low permeability of the overcoating for gas such as water vapour(moisture) and/or oxygen, polymeric films having low permeability for gases based on polymers such as aminoalkyl methacrylate copolymers, sodium carboxymethyl cellulose, hydropropylmethyl cellulose, polyvinyl alcohol can be used. Further inactive ingredients selected from anti-tacking agents, fillers such as microcrystalline cellulose, starch and its derivatives, colorants, pigments, plasticizers can be used in the composition of the overcoating. Overcoating can have an average thickness 5 to 100 µm, preferably 10 to 70 µm and can represent 1 to 15 weight%, preferably 2 to 10 weight% of total mass of coated particles.

All coating steps can be done continously using single pellet coater such as Wurster fluid bed coater, Hüttlin discyet coated, Inoyet coater, where coating and drying of pellets are done in parallel. In case that the residual moisture of obtained coated particles is above 2.5 weight% determined by loss on drying (Mettler Toledo halogen dryer HR73 at 85°C for 20 min.)) additional drying step using the same equipment as for coating step can be used.

Curing step of the coated particles can optionally be used after applying prolonged release coating onto the core particles. Curing can be performed either in coating equipment or by keeping coated particles in stationary phase at elevated temperature in equipment such as drying chamber. Curing is performed at temperatures in the range 30 to 70°C, preferably 35 to 60 °C and most preferably 40 to 60°C. Curing time can be in the range 10 minutes to 24 hours depending on the temperature of the coated particles, relative humidity of the surrounding air and residual moisture in the prolonged release coating. In a special embodiment of the present invention curing can be facilitated by performing curing step in pellet coater by keeping coated particles in low fluidization in order to decrease mechanical damage of the prolonged release coating, with simultaneous spraying of water to increase the moisture content in the prolonged release coating and thus shorten the curing time to less than 2 hours, preferably to less than 1 hour.

### 8. Pharmaceutical Formulation

Coated particles having prolonged release of active substance can be formulated into prolonged release pharmaceutical composition such as a tablet, a capsule or a sachet by the state of the art processes such as compression into tablets, or capsule filling. In order to optimize the process of manufacturing and the properties of the pharmaceutical composition, coated particles can be preferably mixed with excipients selected from diluents, disintegrants, binders, glidants, lubricants, surfactants, antioxidants and optionally flavoring and/or sweetening agents. In case pellets are compressed into tablets it is preferred that diameter of pellets is below 1 mm, preferably below 0.8mm and most preferably below 0.7mm. Excipients in powder form can be admixed with coated prolonged release particles. Homogenous mixture of pellets and excipients can be prepared in mixers known from the state of the art such as biconical or cubic mixer, low shear mixer. The obtained homogenous mixture is compressed into tablets. Obtained tablets can optionally be coated with water soluble film coating which improves organoleptical properties such as taste, colour and/or physical properties of the tablets such as decreased permeability for moisture and/or oxygen. In a special embodiment of the present invention at least 25 weight%, preferably at least 30 weight% of excipients in powder form can be granulated before mixing with pellets. Granulate is preferably made by granulating diluents having plastic deformation properties such as different grade of microcrystalline cellulose with a binder containing liquid. Binder containing liquid can be prepared by dissolving a solid binder such as hydrophilic polymers selected from polyethylene glycols with molecular weight below 10,000, hydropropylmethyl cellulose having viscosity of 2% aqueous solution at 25°C below 20mPas, povidone with K values below 100, copovidone and the like. Tablets containing prolonged release coated particles of the present invention can in one aspect of present invention disintegrate after ingestion by human person in stomach releasing coated prolonged release pellets. Tablets are in such case characterized by in vitro disintegration time measured in water using disintegration test according to the monograph in European Pharmacopeia (6^{th} Ed.) in less than 15 minutes, preferably in less than 10 minutes. In another aspect of the present invention the tablets made according to present invention can be used as orally disintegrating dosage form, which upon contact with saliva disintegrate in mouth releasing coated prolonged release particles containing active substance. In such case it is preferred that disintegrating time of tablets is less than 3 min., preferably less than 1 min.

Disintegrant can be selected from the group of starch, starch derivatives, such as sodium starch glycolate and pregelatinized starch, low-substituted hydroxypropyl cellulose, cross-linked polyvinyl pyrrolidone (crospovidone), polacrilin potassium, and croscarmelose sodium. The disintegrant is preferably cross-linked polyvinyl pyrrolidone.

In a preferred embodiment, the composition can comprise 0.5 to 15 weight%, preferably 1 to 13 weight % of disintegrant based on the weight of the tablet.

Suitable lubricants can be selected from magnesium stearate, magnesium lauryl sulfate, sodium stearyl fumarate, hydrogenated vegetable oil, fatty acid, and stearic acid.

A suitable glidant can be silica.

Sweeteners can be selected from acesulfame K, sucralose, alitame, aspartame, saccharin sodium, dipotassium glycyrrhizinate, stevia and thaumatin.

Flavouring agents can be selected from natural or synthetic materials, such as orange, spearmint, strawberry and cream flavour and the like.

Tablets according to present invention can optionally contain additionally organic acids such as citric acid or tartaric acid.

It is preferred that the tableting excipients include at least one of mannitol, lactose and starch and in particular include all of them.

In order to optimize the release kinetics of active substance from pharmaceutical composition particles with different composition and/or manufacturing procedure resulting in differences in active substance release rate can be mixed before incorporating into pharmaceutical composition.

### 9. Packaging

Low gas permeable primary packaging materials such as aluminium or polychloro-3-fluoroethylene homopolymer/PVC laminate can be used with the thickness in the range 10 to 40 µm in case of Al/Al blisters and 10 to 110 µm in case of Al-polychloro-3-fluoroethylene homopolymer/PVC laminate blisters. Optionally, dosage forms containing paliperidone can be packed into primary packaging with desiccant. Desiccant can be placed inside the packaging unit together with dosage units such as for example tablets and/or in the closure system or can be incorporated into the walls of the primary packaging unit.

To avoid the potential oxidative degradation of incorporated active substance and inactive ingredients susceptible to oxidative degradation, the final dosage form can be packed in primary packaging under inert atmosphere such as for example nitrogen, argon or xenon resulting in decreased concentration of oxygen in the atmosphere surrounding the dosage form in primary packaging such as for example blisters, strips, plastic or glass containers. Decreased concentration of oxygen means, that the concentration of residual oxygen in the atmosphere surrounding the individual dosage form such as for example tablet or capsule is below 10 vol/vol%, preferably below 7.5 vol/vol%, more preferably below 5 vol/vol% and most preferably below 2.5 vol/vol%.

The following examples are to further illustrate preferred aspects of the invention without limiting it thereto.

### 10. Examples

Examples 1-7: composition of core (quantaties for single dose containing 3 or 6 mg of paliperidone)

| Neutral pellets (25 to 35 Mesh) 80 mg | | | | | | | |
|---|---|---|---|---|---|---|---|
| | Example 1 (mg) | Example 2 (mg) | Example 3 (mg) | Example 4 (mg) | Example 5 (mg) | Example 6 (mg) | Example 7 (mg) |
| Inert core: | | | | | | | |
| Neutral pellets | 80 | 80 | 80 | 80 | 300 | 300 | 300 |
| | | | | | | | |

| Active layer: | | | | | | | |
|---|---|---|---|---|---|---|---|
| Paliperidone | 6 | 6 | 6 | 6 | 3 | 3 | 3 |
| Hypromelose 6 mPas | 10 | 10 | 10 | 10 | / | / | / |
| Povidone K30 | / | / | / | / | 5 | 5 | 5.4 |
| Sodium lauryl sulphate | 0.6 | 0.6 | 0.6 | / | 0.27 | 0.27 | 0.27 |
| Sucrose | 23.4 | / | / | / | / | / | / |
| Calcium hydrogen phosphate | / | 23.4 | / | / | / | / | / |
| Talc | / | / | / | / | / | / | 3.75 |
| | | | | | | | |

| Intermediate coating: | | | | | | | |
|---|---|---|---|---|---|---|---|
| Hyprolose | 12 | 10 | / | / | / | / | / |
| Hypromelose 3 mPas | / | / | 10 | / | / | / | / |
| Povidone K25 | / | / | / | 15 | / | / | / |
| Talc | 7 | 5 | 5 | 5 | / | / | / |
| | | | | | | | |

| Prolonged release coating: | | | | | | | |
|---|---|---|---|---|---|---|---|
| Ethylcellulose 45 cp | 30 | / | / | / | / | 46.2 | / |
| Eudragit NE 30 D* | / | 40 | / | / | 46.2 | / | / |
| Kollicoat SR 30 D* | / | / | 50 | 50 | / | / | 46.2 |
| Hypromelose 3 mPas | 3 | 1 | / | / | / | / | / |
| Dibutylsebacate | 2.5 | / | / | / | / | 4.62 | / |
| Triethyl citrate | / | / | 2 | 1.5 | 3.87 | / | 3.87 |
| Talc | 10 | 10 | 10 | 10 | 13.73 | / | 13.73 |

| | | | | | | | |
|---|---|---|---|---|---|---|---|
| * ... dry substance | | | | | | | |

### Procedure for Example 1:

**Phase I: Active layer:** Step 1: Binder (Hypromelose) and sodium lauryl sulphate were dissolved in water. Step 2: To the obtained solution of binder and sodium lauryl sulphate sucrose was added and dissolved. Step 3: Paliperidone was homogenously suspended in the solution obtained in step 2.

The obtained dispersion was sprayed onto neutral pellets on laboratory pellet coater Glatt GPCG-3 with product temperature in the range 30-50°C. The obtained pellets were dried in the coater until the loss of drying (Metller Toledo halogen dryer HR73 at 85°C for 20 min.) was <1.5 weight %. Dried pellets were sieved and transferred back to Glatt GPCG-3 and coated with intermediate coating.

**Phase II: Intermediate coating:** Step 1: Talc was homogenously suspended in part of water. Step 2: Hyprolose was dissolved in water. Step 3: suspension obtained under step I was added to dispersion obtained in step 2 while stirring.

Pellets obtained in Phase I were coated with dispersion obtained in step 3, keeping the product temperature in the range of 30-50 °C. The obtained pellets were dried in the coater until the loss of drying (Metller Toledo halogen dryer HR73 at 85°C for 20 min.) was <1.5 weight %. Dried pellets were sieved and transferred back to Glatt GPCG-3 and coated with prolonged release coating.

**Phase III: Prolonged release coating:** Step 1: Ethylcellulose was dissolved in part of ethanol. Step 2: Hypromelose was dissolved in part of ethanol. Step 3: Dispersion of hypromelose was added to dispersion of ethylcellulose while stirring. Step 4: Talc was homogenously suspended in part of ethanol. Obtained suspension was added to the dispersion obtained in Step 3. Step 5: To the dispersion obtained in Step 4 dibutylsebacate was added. The obtained dispersion was stirred for 60 minutes before coating.

Pellets obtained in Phase II were coated with dispersion obtained in Step 4, keeping the product temperature in the range of 35 to 55°C. After coating pellets were dried briefly in the coater and cooled bellow 30 °C in the coater. Cooled pellets were sieved.

### Procedure for Example 2:

**Phase I: Active layer:** Step 1: Binder (Hypromelose) and sodium lauryl sulphate were dissolved in part of water. Step 2: Calcium hydrogen phosphate was dissolved in part of water. Obtained solution was added to dispersion obtained in step 1. Step 3: Paliperidone was homogenously suspended in the solution obtained in step 2.

The obtained dispersion was sprayed onto neutral pellets on laboratory pellet coater Glatt GPCG-3 with product temperature in the range 30-50°C. The obtained pellets were dried in the coater until the loss of drying (Mettler Toledo halogen dryer HR73 at 85°C for 20 min.) was <1.5 weight %. Dried pellets were sieved and transferred back to Glatt GPCG-3 and coated with intermediate coating.

**Phase II: Intermediate coating:** Step 1: Talc was homogenously suspended in part of water. Step 2: Hyprolose was dissolved in water. Step 3: Suspension obtained under step 1 was added to dispersion obtained in step 2 while stirring.

Pellets obtained in Phase I were coated with dispersion obtained in step 3, keeping the product temperature in the range of 30-50 °C. The obtained pellets were dried in the coater until the loss of drying (Mettler Toledo halogen dryer HR73 at 85°C for 20 min.) was <1.5 weight %. Dried pellets were sieved and transferred back to Glatt GPCG-3 and coated with prolonged release coating.

**Phase III: Prolonged release coating:** Step 1: Talc was homogenously suspended in part of water. Step 2: Hypromelose was dispersed in part of water. Step 3: Suspension obtained in Step 1 and dispersion obtained in Step 2 were added to Eudragit NE 30 D dispersion while stirring.

Pellets obtained in Phase II were coated with dispersion obtained in step 3, keeping the product temperature in the range of 20 to 25°C. After coating pellets were dried for 15 min in the coater at product temperature 40°C with minimal fluidization of pellets and cooled to 25°C in the coater. Cooled pellets were sieved.

### Procedure for Example 3:

**Phase I: Active layer:** Step 1: Binder (Hypromelose) and sodium lauryl sulphate were dissolved in water. Step 2: Paliperidone was homogenously suspended in the solution obtained in Step 1.

The obtained dispersion was sprayed onto neutral pellets on laboratory pellet coater Glatt GPCG-3 with product temperature in the range 30-50°C. The obtained pellets were dried in the coater until the loss of drying (Mettler Toledo halogen dryer HR73 at 85°C for 20 min.) was <1.5 weight %. Dried pellets were sieved and transferred back to Glatt GPCG-3 and coated with intermediate coating.

**Phase II: Intermediate coating:** Step 1: Talc was homogenously suspended in part of water. Step 2: Hypromelose was dissolved in water. Step 3: Suspension obtained under step 1 was added to dispersion obtained in step 2 while stirring.

Pellets obtained in Phase I were coated with dispersion obtained in step 3, keeping the product temperature in the range of 30-50°C. The obtained pellets were dried in the coater until the loss of drying (Mettler Toledo halogen dryer HR73 at 85°C for 20 min.) was <1.5 weight %. Dried pellets were sieved and transferred back to Glatt GPCG-3 and coated with prolonged release coating.

**Phase III: Prolonged release coating:** Step 1: Talc was homogenously suspended in part of water. Step 2: Triethyl citrate was dispersed in part of water and Kollicoat SR 30D was added to the obtained dispersion. Step 3: Suspension obtained under Step 1 was added to dispersion obtained in Step 2 while stirring.

Pellets obtained in Phase II were coated with dispersion obtained in step 3, keeping the product temperature in the range of 35 to 40°C. After coating pellets were dried for 15 min in the coater at product temperature 40°C with minimal fluidization of pellets and cooled to 25°C in the coater. Cooled pellets were sieved.

### Procedure for Example 4:

**Phase I: Active layer:** Step 1: Binder (Hypromelose) was dissolved in water. Step 2: Paliperidone was homogenously suspended in the solution obtained in Step 1.

The obtained dispersion was sprayed onto neutral pellets on laboratory pellet coater Glatt GPCG-3 with product temperature in the range 30-50°C. The obtained pellets were dried in the coater until the loss of drying (Mettler Toledo halogen dryer HR73 at 85°C for 20 min.) was <1.5 weight %. Dried pellets were sieved and transferred back to Glatt GPCG-3 and coated with intermediate coating.

**Phase II: Intermediate coating:** Step 1: Talc was homogenously suspended in part of water. Step 2: Povidone K25 was dissolved in water. Step 3: Suspension obtained under step 1 was added to dispersion obtained in step 2 while stirring.

Pellets obtained in Phase I were coated with dispersion obtained in step 3, keeping the product temperature in the range of 30-50°C. The obtained pellets were dried in the coater until the loss of drying (Mettler Toledo halogen dryer HR73 at 85°C for 20 min.) was <1.5 weight %. Dried pellets were sieved and transferred back to Glatt GPCG-3 and coated with prolonged release coating.

**Phase III: Prolonged release coating:** Step 1: Talc was homogenously suspended in part of water. Step 2: Triethyl citrate was dispersed in water and Kollicoat SR 30D was added to the obtained dispersion. Step 3: Suspension obtained under Step 1 was added to dispersion obtained in Step 2 while stirring.

Pellets obtained in Phase II were coated with dispersion obtained in step 3, keeping the product temperature in the range of 35 to 40°C. After coating pellets were dried for 15 min in the coater at product temperature 40°C with minimal fluidization of pellets and cooled to 25°C in the coater. Cooled pellets were sieved.

### Procedure for Example 5:

**Phase I: Active layer:** Step 1: Binder (Povidone K30) and sodium lauryl sulphate were dissolved in water. Step 2: Paliperidone was homogenously suspended in the solution obtained in step 1.

The obtained dispersion was sprayed onto neutral pellets on laboratory pellet coater Glatt GPCG-3 with product temperature in the range 30-50°C. The obtained pellets were dried in the coater until the loss of drying (Mettler Toledo halogen dryer HR73 at 85°C for 20 min.) was <1.5 weight %. Dried pellets were sieved and transferred back to Glatt GPCG-3 and coated with prolonged release coating.

**Phase II: Prolonged release coating:** Step 1: Talc and triethyl citrate were homogenously dispersed in part of water. Step 2: Dispersion obtained in step 1 was added to Eudragit NE 30 dispersion while stirring.

Pellets obtained in Phase I were coated with dispersion obtained in step 2 in the fluid bed coater, keeping the product temperature in the range of 20 to 25°C. After coating pellets were cured in same coater at 40°C for 30 minutes with slow spraying of water (less than 1 g/min/kg). After curing step was completed, pellets were dried in same equipment at product temperature 35-40°C until the residual moisture of pellets was below 1.6 weight% (determined by Mettler Toledo halogen dryer HR73 at 85°C for 20 min. and cooled to 25°C in the coater. Cooled pellets were sieved.

### Procedure for Example 6:

**Phase I: Active layer**: Step 1: Binder (Povidone K30) and sodium lauryl sulphate were dissolved in water. Step 2: Paliperidone was homogenously suspended in the solution obtained in step 1.

The obtained dispersion was sprayed onto neutral pellets on laboratory pellet coater Glatt GPCG-3 with product temperature in the range 30-50°C. The obtained pellets were dried in the coater until the loss of drying (Mettler Toledo halogen dryer HR73 at 85°C for 20 min.) was <1.5 weight %. Dried pellets were sieved and transferred back to Glatt GPCG-3 and coated with prolonged release coating.

**Phase II: Prolonged release coating**: Step 1: Ethylcellulose was dissolved in ethanol. Step 2: To the dispersion obtained in Step 1 dibutylsebacate was added. The obtained dispersion was stirred for 60 minutes before coating.

Pellets obtained in Phase I were coated with dispersion obtained in Step 2, keeping the product temperature in the range of 35 to 55°C. After coating pellets were dried briefly in the coater and cooled bellow 30°C in the coater. Cooled pellets were sieved.

### Procedure for Example 7:

**Phase I: Active layer**: Step 1: Binder (Povidone K30) and sodium lauryl sulphate were dissolved in water. Step 2: Paliperidone was homogenously suspended in the solution obtained in step 1. Step 3: A suspension of talc in water was prepared separately and admixed to the dispersion obtained in step 2.

The obtained dispersion was sprayed onto neutral pellets on laboratory pellet coater Glatt GPCG-3 with product temperature in the range 30-50°C. The obtained pellets were dried in the coater until the loss of drying (Mettler Toledo halogen dryer HR73 at 85°C for 20 min.) was <1.5 weight %. The dried pellets were sieved and transferred back to Glatt GPCG-3 and coated with prolonged release coating.

**Phase II: Prolonged release coating:** Step 1: Talc was homogenously suspended in part of water. Step 2: Triethyl citrate was dispersed in water and Kollicoat SR 30D was added to the obtained dispersion. Step 3: Suspension obtained under step 1 was added to dispersion obtained in step 2 while stirring.

Pellets obtained in Phase I were coated with dispersion obtained in step 3, keeping the product temperature in the range of 35 to 40°C. After coating pellets were dried for 15 min in the coater at product temperature 38-40°C with minimal fluidization of pellets and cooled to 25°C in the coater. Cooled pellets were sieved. Sieved pellets were transferred to the plates with thickness of pellets layer on the plate less than 3cm. The plates with pellets were transferred to drying chamber and kept at 40°C for 8 hours. Pellets were cooled down to room temperature after completion of drying step and packed into polyethylene bags.

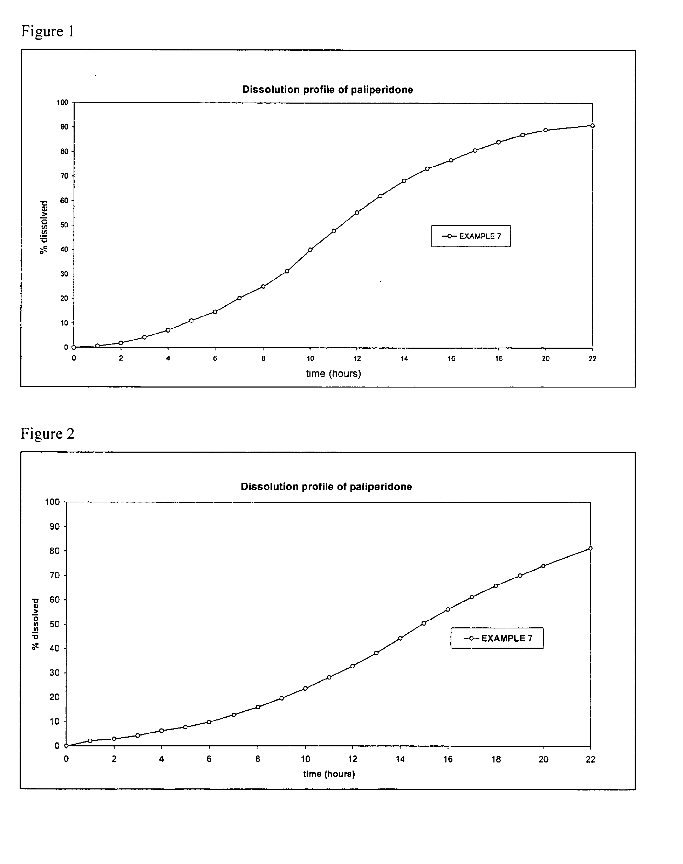

Example 7 exhibits an *in vitro* dissolution profile such that after 2 hours less than 25% of the active substance originally contained in a particle type is released, after 12 hours between 25% and 65% of the active substance originally contained in a particle type is released, and after 24 hours more than 65% of the active substance is released. This is illustrated by Figure 1.

Example 7 exhibits an *in vitro* dissolution profile such that after 2 hours less than 25% of the active substance originally contained in a particle type is released, after 12 hours between 25% and 65% of the active substance originally contained in a particle type is released, and after 24 hours more than 65% of the active substance originally contained in a particle type is released in USP Buffer pH 6.8 at 37°C in an Apparatus 1 - basket (Ph.Eur. or USP, 100 rpm). The dissolution characteristics of the formulation of Example 7 under these conditions are shown in the appended Figure 2.

### Examples 8-12: Pharmaceutical compositions with 1.5 to 15 mg of paliperidone

| | Example 8 (mg) | Example 9 (mg) | Example 10 (mg) | Example 11 (mg) | Example 12 (mg) |
|---|---|---|---|---|---|
| Inert core: | | | | | |
| Neutral pellets | 80.0 | 60.0 | 360.0 | 300.0 | 300.0 |
| | | | | | |

| Active layer: | | | | | |
|---|---|---|---|---|---|
| Paliperidone | 1.5 | 1.5 | 9.0 | 9.0 | 15 |
| Hypromelose 3 mPas | 10 | 6 | 36.0 | / | 15 |
| Povidone K30 | / | / | / | 15.0 | / |
| Sodium lauryl sulphate | 0.2 | 0.15 | 0.9 | 0.9 | 1.5 |
| Mannitol | 25.0 | 10.35 | 62.4 | / | 40.0 |
| Talc | / | / | / | 9.1 | / |
| | | | | | |

| Intermediate coating: | | | | | |
|---|---|---|---|---|---|
| Hyprolose | 12 | 8 | 48.0 | / | / |
| Hypromelose 3 mPas | / | / | / | / | 50 |
| Povidone K25 | / | / | / | / | / |
| Talc | 7 | 5 | 30.0 | / | 25 |
| | | | | | |

| Prolonged release coating: | | | | | |
|---|---|---|---|---|---|
| Ethylcellulose 45 cp | 30 | / | / | 46.2 | / |
| Eudragit NE 30 D* | / | 25 | 150.0 | / | / |
| Kollicoat SR 30 D* | / | / | / | / | 75 |
| Hypromelose 3 mPas | 3 | 1 | 6.0 | / | / |
| Dibutylsebacate | 2.5 | / | / | 4.62 | / |
| Triethyl citrate | / | / | / | / | 3 |
| Talc | 10 | 7 | 42.0 | / | 22 |

| | | | | | |
|---|---|---|---|---|---|
| * ... dry substance | | | | | |

Examples 8-12 were prepared analogously to the processes described for Examples 1-7.

### Examples 13 and 14 - composition for 10,000 capsules

| | Example 13 | Example 14 | Example 15 |
|---|---|---|---|
| Pellets from Example 7 | 3,762.20 g | 3,762.20 g | / |
| Pellets from Example 11 | / | / | 3,848.20 g |
| Talc | 7.52 g | 7.52 g | / |
| Aerosil 200 | / | 3.76 g | 3.85 g |

Pellets are mixed with talc or with talc and Aerosil and filled into capsules.

## Claims

1. Pharmaceutical composition comprising paliperidone or a pharmaceutically acceptable salt thereof as the active substance, wherein the pharmaceutical composition is in the form of pellets comprising a core particle, optionally one or more intermediate coatings, at least one prolonged release coating and optionally one or more outer coatings, in this order,
wherein the active substance is present in the core particle, and
wherein the pellets have a maximum diameter of 2 mm or less and exhibit the following release characteristics when being submerged in USP buffer pH 6.8 at 37°C in an Apparatus 1 - basket (Ph. Eur. or USP, 100 rpm):
(a) Release of < 25 wt.-% of active substance at 2 hours after beginning of the experiment;
(b) Release of 25-65wt.-% of active substance at 12 hours after beginning of the experiment;
(c) Release of >65wt.-% of active substance at 24 hours after beginning of the experiment,
all indications of wt.% being based on the weight of the entire pharmaceutical composition.

2. Pharmaceutical composition according to Claim 1, wherein the maximum diameter of the pellets is 1.4 mm or less.

3. Pharmaceutical composition according to Claim 1 or 2, wherein the prolonged release coating comprises a prolonged release substance in the weight ratio to the total weight of coated spherical particles in the range from 1 : 5 to 1 : 50, preferably from 1:7 to 1:30

4. Pharmaceutical composition according to any one of Claims 1 to 3, wherein the prolonged release coating has an average thickness within the range of from 10 to 80 µm.

5. Pharmaceutical composition according to any one of Claims 1 to 4, wherein the prolonged release substance is selected from excipients that swell upon contact with physiological fluids, non-swellable polymers, and insoluble excipients.

6. Pharmaceutical composition according to any one of Claims 1 to 5, wherein the release kinetics are essentially not dependent on the pH of the medium.

7. Pharmaceutical composition according to any one of Claims 1 to 6, wherein none of the optionally present outer layers contains a polymer that is in ionized form at neutral pH.

8. Pharmaceutical composition according to any one of Claims 1 to 7, wherein the core particles are selected from matrix core particles, core particles obtained from melt pelletization, and core particles obtained by coating an inert particles with a layer comprising the active substance.

9. Pharmaceutical composition according to any one of Claims 1 to 8 for use in a method for treating or preventing schizophrenia.

10. Use of a pharmaceutical composition according to any one of Claims 1 to 8, in a method of manufacturing a medicament for the treatment or prophylaxis of schizophrenia.

11. Method for preparing a pharmaceutical composition according to any one of Claims 1 to 8, comprising the step of coating particles containing the active substance with a prolonged release coating.
